(19) Europäisches Patentamt
European Patent Office
Office européen des brevets

(11) **EP 3 071 323 B1**

(12) **FASCICULE DE BREVET EUROPEEN**

(45) Date de publication et mention de la délivrance du brevet:
**03.04.2024 Bulletin 2024/14**

(21) Numéro de dépôt: **14800077.1**

(22) Date de dépôt: **20.11.2014**

(51) Classification Internationale des Brevets (IPC):
**B01J 20/28** (2006.01)  **B01J 20/12** (2006.01)
**B01J 20/18** (2006.01)  **B01J 20/30** (2006.01)
**C07C 7/13** (2006.01)

(52) Classification Coopérative des Brevets (CPC):
(C-Sets disponibles)
**C07C 7/13; B01J 20/12; B01J 20/18; B01J 20/183;
B01J 20/28004; B01J 20/28011; B01J 20/28016;
B01J 20/28069; B01J 20/2808; B01J 20/28083;
B01J 20/28085; B01J 20/28088; B01J 20/28092;
B01J 20/3042; B01J 20/3078;** (Cont.)

(86) Numéro de dépôt international:
**PCT/EP2014/075191**

(87) Numéro de publication internationale:
**WO 2015/075140 (28.05.2015 Gazette 2015/21)**

(54) **MATÉRIAU GRANULAIRE ZÉOLITHIQUE À STRUCTURE CONNEXE**

KÖRNIGES ZEOLITHMATERIAL MIT EINER VERBUNDENEN STRUKTUR

ZEOLITIC GRANULAR MATERIAL HAVING A CONNECTED STRUCTURE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.11.2013 FR 1361380**

(43) Date de publication de la demande:
**28.09.2016 Bulletin 2016/39**

(73) Titulaires:
• **ARKEMA FRANCE**
**92700 Colombes (FR)**
• **IFP Energies nouvelles**
**92500 Rueil-Malmaison (FR)**

(72) Inventeurs:
• **LAROCHE, Catherine**
**F-69390 Vernaison (FR)**
• **BOUVIER, Ludivine**
**F-64300 Orthez (FR)**
• **LEFLAIVE, Philibert**
**F-69780 Mions (FR)**
• **LUTZ, Cécile**
**F-64290 Gan (FR)**
• **GAY, Anne-Sophie**
**F-42100 Saint-Etienne (FR)**
• **MOREAU, Florent**
**F-69003 Lyon (FR)**

(74) Mandataire: **Bandpay & Greuter**
**11 rue Christophe Colomb**
**75008 Paris (FR)**

(56) Documents cités:
**WO-A1-2008/009845   WO-A1-2014/177567
WO-A2-2008/152319   WO-A2-2009/081023
US-A1- 2011 104 494   US-A1- 2011 105 301
US-A1- 2013 012 377**

• **Ivan Petrov ET AL: "Synthesis of Zeolite A: A Review", , 1 January 2012 (2012-01-01), XP055693902, Retrieved from the Internet: URL:https://pdfs.semanticscholar.org/080d/ 64892e31cef52edac82141f8a4c6a08440ac.pdf?_ ga=2.224899162.1572276088.1589262219-64982 6876.1589262219 [retrieved on 2020-05-12]**

(52) Classification Coopérative des Brevets (CPC):
(Cont.)
**B01J 20/3085**

C-Sets
**C07C 7/13, C07C 15/08**

**Description**

La présente invention concerne le domaine des adsorbants à structure zéolithique et plus particulièrement le domaine des adsorbants à structure zéolithique sous forme d'agglomérés. La présente invention concerne également la synthèse desdits agglomérés à structure zéolithique, ainsi que leurs utilisations pour la séparation de mélanges gazeux ou liquides.

La synthèse de zéolithes conduit à des cristaux, généralement sous forme de poudre, dont l'emploi à l'échelle industrielle est particulièrement malaisé. En effet, on sait aujourd'hui synthétiser des cristaux de zéolithe de taille variant de quelques nanomètres à quelques micromètres, tailles requises pour conférer aux zéolithes des capacités optimales d'adsorption. Les inconvénients liés à ces cristaux de petites tailles sont cependant nombreux, inconvénients parmi lesquels on peut citer la difficulté de manutention de poudre pulvérulente et les pertes de charge importantes lors de leurs utilisations.

Afin de pallier ces inconvénients, il a donc été proposé d'utiliser des formes agglomérées de ces cristaux, par exemple sous forme de grains, de filés, de billes et autres formes agglomérées. La fabrication de tels agglomérés à partir de cristaux de zéolithe(s) sous forme de poudre est bien connue aujourd'hui, et la littérature scientifique et la littérature brevet fournissent de nombreux exemples de préparation d'agglomérés zéolithiques, notamment par extrusion, pastillage, et autres techniques d'agglomération connues de l'homme du métier.

Ces agglomérés sont habituellement de tailles de l'ordre de quelques dizaines de micromètres, voire de quelques centaines de micromètres, voire de quelques millimètres, et ne présentent pas les inconvénients inhérents aux matières pulvérulentes que sont les cristaux de zéolithes précédemment définis.

Ces agglomérés, qu'ils soient sous forme de plaquettes, de billes, d'extrudés, et autres, sont en général constitués de cristaux de zéolithe(s), qui constituent l'élément actif (au sens de l'adsorption) et d'un liant d'agglomération.

Ce liant d'agglomération est destiné à assurer la cohésion des cristaux entre eux dans la structure agglomérée, mais aussi doit permettre d'assurer une résistance mécanique suffisante auxdits agglomérés afin d'éviter, ou tout au moins de minimiser le plus possible, les risques de fractures, brisures ou cassures qui pourraient survenir lors de leurs utilisations industrielles pendant lesquelles les agglomérés sont soumis à de nombreuses contraintes, telles que vibrations, variations fortes et/ou fréquentes de pression, mouvements et autres. Il est donc très important que les agglomérés zéolithiques soumis à ces diverses contraintes restent cohésifs et ne puissent pas générer de fines particules pulvérulentes conduisant aux inconvénients précités.

Par ailleurs, les facteurs importants qui influencent les performances d'un procédé de séparation par adsorption englobent notamment la sélectivité d'adsorption, la capacité d'adsorption et la cinétique de transfert de matière qui définit les vitesses d'adsorption et de désorption des différents composés. L'adsorbant doit donc présenter de bonnes propriétés de transfert de matière afin de garantir un nombre de plateaux théoriques suffisants pour réaliser une séparation efficace des espèces en mélange, comme l'indique Ruthven dans l'ouvrage intitulé « Principles of Adsorption and Adsorption Processes » (« Principes de l'Adsorption et des Procédés d'Adsorption »), John Wiley & Sons, (1984), pages 326 et 407.

Dans le cas d'un adsorbant zéolithique sous forme d'aggloméré, le transfert de matière global dépend de l'addition de la résistance diffusionnelle intra-cristalline et de la résistance diffusionnelle entre les cristaux (voir Ruthven, *ibid.* p. 243). La résistance diffusionnelle intra-cristalline est proportionnelle au carré des rayons des cristaux et inversement proportionnelle à la diffusivité intra-cristalline des molécules à séparer par adsorption. La résistance diffusionnelle entre les cristaux (également appelée résistance macroporeuse) est quant à elle proportionnelle au carré des rayons des agglomérés et inversement proportionnelle à la diffusivité des molécules à séparer dans les macropores.

On cherche ainsi à réduire le plus possible le diamètre des cristaux de zéolithes et/ou réduire le plus possible le diamètre des agglomérés afin d'améliorer le transfert de matière. De tels agglomérés de taille réduite, comportant éventuellement des cristaux de taille réduite, s'avèrent cependant plus fragiles, et il devient alors nécessaire d'augmenter le taux de liant d'agglomération afin de renforcer la cohésion des cristaux entre eux au sein de l'aggloméré. Toutefois, l'augmentation du taux de liant d'agglomération, inerte vis-à-vis de l'adsorption, réduit d'autant les capacités d'adsorption des agglomérés par rapport à des cristaux de zéolithe(s) utilisés seuls, sous forme de poudre non agglomérée.

Un besoin est donc apparu de créer des adsorbants zéolithiques sous forme d'agglomérés combinant de bonnes propriétés de transfert de matière, une dimension compatible avec son utilisation à l'échelle industrielle, typiquement entre 0,1 mm et 1 mm, et une très grande résistance mécanique, tout en conservant les propriétés d'adsorption nécessaires à leurs utilisations.

L'art antérieur propose plusieurs possibilités, parmi lesquelles une première possibilité consiste en la réalisation de monocristaux de zéolithe de taille suffisamment importante pour être directement manipulables dans un procédé de séparation industriel. La réalisation de monocristaux de zéolithe de grande dimension a été discutée dans la littérature. En particulier Qiu et coll. (Micropor. Mesopor. Mater., 21 (4-6), (1998), 245-251,) indiquent que des monocristaux de zéolithe de structure faujasite de taille allant jusqu'à 150 $\mu$m peuvent être obtenus en présence d'inhibiteurs de nucléation et en utilisant des réactifs de très haute pureté. Outre les difficultés prévisibles pour l'industrialisation de ce type de synthèse, il semble impossible de synthétiser des cristaux de zéolithes de taille comprise entre 0,1 mm et 1 mm qui serait typiquement la taille requise pour une utilisation dans un procédé industriel, notamment en phase liquide, sans difficulté majeure de mise en oeuvre. De plus, la macroporosité et la mésoporosité de ces matériaux étant quasiment nulles, il semble très probable d'observer un très mauvais transport des composés à adsorber/séparer au sein de la phase zéolithique sur une distance caractéristique correspondant à la taille de l'aggloméré, ou de l'adsorbant zéolithique sous forme de monolithe cristallin.

Les demandes de brevets US2012/0093715 et US2013/0052126 enseignent que l'on peut former des structures zéolithiques monolithiques avec une structure à porosité hiérarchisée. La méthode proposée consiste à former un gel de précurseur de zéolithe par hydrolyse. Ce gel est chauffé à une température suffisamment élevée et pendant suffisamment longtemps pour cristalliser et agglomérer le gel en une structure zéolithique monolithique. L'addition d'un polymère au milieu réactionnel conduit à l'obtention de structures zéolithiques monolithiques avec une structure ayant une porosité hiérarchisée.

Néanmoins, les structures zéolithiques monolithiques décrites dans ces demandes de brevets présentent une macroporosité et une mésoporosité, mesurée par intrusion au mercure, faible dans le cas où aucun structurant organique n'est utilisé. On peut donc craindre que, comme dans le cas des monocristaux, le transfert de matière au sein de la structure soit alors relativement médiocre. Dans le cas où un agent organique polymère est utilisé, et outre le fait qu'une étape de retrait de l'agent organique est nécessaire, le solide présente au contraire une porosité très élevée.

On peut alors craindre que la résistance mécanique du matériau soit peu élevée et que la capacité d'adsorption par unité de volume de lit d'adsorbant soit également faible.

La demande WO2000/000287, décrit également l'obtention de macrostructures inorganiques dont la porosité est créée et contrôlée par l'utilisation d'une résine organique qui doit être éliminée en fin de procédé.

L'ensemble des demandes ci-dessus apparaissent en outre peu économiques, car les procédés explicités nécessitent l'emploi d'agents organiques qui impliquent un coût et une étape d'élimination supplémentaires lors du procédé de fabrication.

L'obtention de monolithes de zéolithe 13X à porosité hiérarchisée est également décrite par Akhtar et Bergström (J. Am. Chem. Soc., 94(1), (2001), 92-98). Les auteurs montrent que le moulage de suspensions colloïdales de poudre de zéolithe 13X concentrées et stabilisées par addition de polyéthylèneglycol permet l'obtention de matériaux dits corps verts ou « green bodies » en langue anglaise, lesquels, après traitement thermique « flash » à 800°C conduisent à des monolithes présentant une porosité hiérarchisée, lesdits monolithes étant résistants et ayant une capacité d'adsorption du $CO_2$ proche de celle de la poudre (cristaux de zéolithes) correspondante.

Ces matériaux de taille relativement importante (la taille caractéristique est indiquée comme étant supérieure à 10 mm) obtenus par le procédé décrit (moulage) semblent pouvoir bien convenir pour des procédés en phase gaz, l'essentiel de la résistance au transfert de matière étant alors dans les micropores. Toutefois, on peut douter que de tels matériaux soient très performants pour des applications en phase liquide ou le transfert de matière dans les macro-mésopores doit également être optimisé.

WO2008/009845 divulgue des adsorbants zéolitiques agglomérés à base de cristaux de zéolite X de taille inférieure à 1,7 $\mu$m, échangés au baryum ou baryum + potassium, à liant zéolithisé et dont la résistance mécanique est supérieure à 2 MPa. WO2009/081023 divulgue un adsorbant zéolitique aggloméré à base de cristaux de zéolite LSX de diamètre moyen en nombre compris entre 0,1 $\mu$m et 4 $\mu$m, échangés au baryum ou baryum + potassium, présentant une résistance supérieure à 2 MPa.

Il reste par conséquent un besoin pour des matériaux adsorbants zéolithiques non pulvérulents, de grande résistance mécanique et dont les capacités d'adsorption en phase gaz et/ou liquide, de préférence en phase liquide, sont grandement améliorées.

Ainsi un premier objectif de la présente invention est de fournir un matériau granulaire zéolithique composé d'une structure zéolithique percolante sur tout le volume du matériau, présentant une grande résistance mécanique, un transfert de matière optimisé dans les macropores et les mésopores, facilement industrialisable et avec des coûts de fabrication peu élevés. D'autres objectifs encore apparaîtront à la lumière de la description de la présente invention qui suit.

Les inventeurs sont parvenus à préparer un tel matériau granulaire zéolithique répondant aux divers objectifs précités et permettant de pallier en totalité ou tout au moins en partie les inconvénients et défauts des adsorbants connus de l'art antérieur.

Ainsi, et selon un premier aspect, la présente invention a pour objet un matériau granulaire zéolithique présentant une résistance mécanique élevée, et une structure zéolithique percolante, c'est-à-dire formant un ensemble connexe sur tout le volume dudit matériau.

L'invention concerne un matériau granulaire zéolithique de petite taille, comprise entre 0,1 mm et 1 mm, bornes incluses, à structure percolante, assurant ainsi un transport optimisé des molécules que l'on souhaite séparer par adsorption, en phase liquide ou en phase gaz, de préférence en phase liquide, au sein des macropores et mésopores du matériau et au sein de la phase zéolithique, et ayant une structure particulière permettant d'obtenir une très haute résistance mécanique.

Le matériau granulaire zéolithique selon la présente invention présente une résistance mécanique à l'écrasement en lit (REL) élevée, mesurée par la méthode Shell série SMS1471-74 adaptée pour des agglomérés de taille inférieure à 1,6 mm, comme décrit plus loin dans la description. La REL du matériau granulaire zéolithique de l'invention est supérieure ou égale à 1,0 MPa, plus généralement supérieure ou égale à 1,5 MPa, de préférence supérieure ou égale à 2,0 MPa, typiquement supérieure ou égale à 2,1 MPa.

Par « ensemble connexe sur tout le volume dudit matériau », on entend une structure tridimensionnellement connexe, et de préférence tridimensionnellement connexe par arcs, c'est-à-dire que tout couple de points de la structure zéolithique est relié par un chemin, le terme « chemin » étant pris dans sa définition mathématique utilisée en topologie, augmentant ainsi la densité du réseau de "chaînes de forces" supportant l'ensemble des contraintes mécaniques.

Pour les besoins de l'invention, on réalise plusieurs sections polies que l'on examine au microscope électronique à balayage (MEB). On réalise 10 clichés sur les différentes sections polies, avec un grossissement compris entre 5000 et 10000, et on évalue par analyse d'images, ou tout autre moyen approprié, la surface formant un ensemble connexe, pour chacun des 10 clichés. Le matériau granulaire zéolithique de l'invention est considéré comme « ensemble connexe sur tout le volume dudit matériau » lorsque la proportion (moyenne des 10 clichés) de surface connexe représente au moins 50%, de préférence au moins 70%, de préférence encore au moins 80% de la surface occupée par le matériau sur chaque cliché.

Selon un aspect préféré de l'invention, le matériau granulaire zéolithique présente une macroporosité ouverte. Par « macroporosité ouverte », on entend une porosité accessible depuis la surface externe du matériau granulaire, une telle porosité pouvant être caractérisée par la technique dite « par intrusion de mercure », technique de porosimétrie bien connue de l'homme du métier.

Dans la description de l'invention, on entend par « taille » le diamètre moyen en nombre d'un objet, ou sa plus grande dimension moyenne en nombre lorsqu'il n'est pas sphérique. Le matériau granulaire zéolithique de l'invention présente une taille comprise entre 0,1 mm et 1 mm, de préférence comprise entre 0,2 mm et 1 mm, et en particulier comprise entre 0,3 mm et 0,8 mm, plus généralement entre 0,4 mm et 0,6 mm, bornes incluses.

Le matériau granulaire zéolithique de l'invention comprend à la fois des macropores, des mésopores et des micropores. Par « macropores », on entend des pores dont l'ouverture est supérieure à 50 nm, de préférence comprise entre 50 nm et 400 nm, de préférence encore comprise entre 50 nm et 300 nm. Par « mésopores », on entend des pores dont l'ouverture est comprise entre 2 nm et 50 nm, bornes non incluses. Par « micropores », on entend des pores dont l'ouverture est inférieure à 2 nm, typiquement supérieure strictement à 0 et inférieure ou égale à 2 nm.

Le matériau granulaire selon la présente invention est un matériau zéolithique, indiquant qu'il comprend au moins une zéolithe choisie parmi les zéolithes de structure FAU, notamment choisie parmi les zéolithes LSX, MSX, X, Y, et parmi les zéolithes de structure EMT, LTA ou MFI. Parmi ces zéolithes, on préfère celles choisies parmi les zéolithes LSX, MSX, X, de préférence encore la zéolithe X, sans exclure les mélanges de zéolithes X avec une ou plusieurs des autres zéolithes listées ci-dessus.

En règle générale, le matériau granulaire zéolithique de l'invention comprend plus de 94% en poids de zéolithe(s), de préférence entre 96% et 98% en poids de zéolithe(s), bornes incluses, par rapport au poids total du matériau granulaire zéolithique.

Selon un mode de réalisation préféré, la (ou les) zéolithe(s) se présentent sous la forme de cristaux de taille généralement comprise entre 10 nm et 1500 nm, de préférence entre 100 nm et 1200 nm, de préférence encore entre 200 nm et 1100 nm, et de manière tout particulièrement préférée entre 400 nm et 1000 nm, bornes incluses.

Le matériau granulaire zéolithique de la présente invention se caractérise en outre par une distribution poreuse spécifique, où les volumes macroporeux et mésoporeux sont mesurés par intrusion de mercure et le volume microporeux est mesuré par adsorption d'azote. Par « distribution poreuse », on entend la répartition du volume poreux en fonction du diamètre des pores. Par ailleurs, dans la description de la présente invention, on désigne par « Vma » le volume macroporeux, par « Vme » le volume mésoporeux et par « Vmi » le volume microporeux. « Vg » représente le volume total du matériau granulaire.

Selon un mode de réalisation tout particulièrement préféré de la présente invention, la distribution poreuse du matériau granulaire zéolithique satisfait aux inéquations 1), 2) et 3) suivantes :

$$1) \quad \frac{Vme}{Vme + Vma} \leq 0{,}1, \quad \text{de préférence} \quad 0{,}01 \leq \frac{Vme}{Vme + Vma} \leq 0{,}1, \quad \text{plus}$$

$$\text{préférentiellement } 0{,}01 \leq \frac{Vme}{Vme + Vma} \leq 0{,}06 \; ;$$

$$2) \quad 0.4 \leq \frac{Vmi}{Vma + Vme + Vmi}, \text{ de préférence } 0.4 \leq \frac{Vmi}{Vma + Vme + Vmi} \leq 0.6,$$

$$\text{de préférence encore } 0.45 \leq \frac{Vmi}{Vma + Vme + Vmi} \leq 0.55 \text{ ; et}$$

$$3) \quad 0.25 \leq \frac{Vma + Vme}{Vg} \leq 0.35.$$

La distribution poreuse spécifique du matériau granulaire zéolithique de l'invention décrit un matériau dont les volumes microporeux, mésoporeux et macroporeux sont ajustés, selon les ratios définis précédemment, de manière à permettre un transport efficace depuis l'extérieur du matériau granulaire vers la structure zéolithique, des composés à séparer par adsorption, tout en conservant une capacité d'adsorption optimale.

La combinaison de cette distribution poreuse spécifique, avec la nature connexe de la structure zéolithique, permet de conférer audit matériau d'une part de très bonnes propriétés de séparations de liquides ou de gaz, et d'autre part une très bonne résistance mécanique, grâce à l'existence de ponts (ou voûtes) entre les cristaux.

Il est en effet connu que lorsqu'on sollicite un ensemble de cristaux, par compression, ou bien en exerçant un cisaillement, la répartition des forces qui en résultent au sein de cet ensemble est très hétérogène. En effet, certains cristaux ne sont pratiquement pas sous contrainte, alors que toute la contrainte repose sur d'autres.

La création d'un assemblage solidaire de cristaux de zéolithes connectés entre eux par des voûtes ou des ponts de même nature, à partir d'un matériau préparé par agglomération de cristaux, augmente la densité du réseau de "chaînes de forces" supportant l'ensemble des contraintes ce qui renforce ainsi considérablement la structure mécanique dudit matériau zéolithique en lui apportant une rigidité proche de celle d'un matériau sans porosité.

Ainsi, le matériau granulaire zéolithique de l'invention comprend une macroporosité et une mésoporosité dans laquelle la phase liquide ou gazeuse circule pour transporter les composés à séparer par adsorption, de l'extérieur du grain vers la microporosité, et inversement.

Cette macroporosité permet un transport efficace depuis l'extérieur vers la surface des micropores et inversement, sans avoir à circuler à travers des pores de dimensions inférieures : i.e. les macropores sont les pores majoritaires pour le transport des composés vers et depuis les micropores.

Selon un autre aspect, l'invention concerne un procédé de préparation des matériaux granulaires zéolithiques tels qu'ils viennent d'être définis, procédé qui comprend au moins les étapes de :

a) mélange de cristaux d'au moins une zéolithe, avec un liant argileux contenant au moins 80%, de préférence au moins 90%, de préférence encore au moins 95% en poids d'argile zéolithisable, et éventuellement une source de silice,

b) mise en contact avec une solution basique alcaline, typiquement une solution d'hydroxyde de sodium ou d'un mélange d'hydroxyde de sodium et d'hydroxyde de potassium,

c) mise en température de la suspension entre 80°C et 600°C, de préférence entre 80°C et 150°C, dans un conteneur clos et étanche, sous une pression au moins égale à la pression autogène, typiquement comprise entre la pression autogène et 20 bars.

d) échange cationique éventuel des cations contenu dans le milieu réactionnel de l'étape c) par mise en contact avec une solution d'ions baryum, ou d'ions baryum et d'ions potassium,

e) lavage et séchage des matériaux granulaires zéolithiques ainsi obtenus, et éventuellement au moins une étape de mise en forme à la taille souhaitée, et

f) activation par chauffage à une température en général comprise entre 100°C et 400°C, de préférence entre 200°C et 300°C du matériau granulaire zéolithique obtenus à l'étape e).

La taille des cristaux de zéolithe(s) à l'étape a) et des cristaux de zéolithe(s) dans les matériaux granulaires est mesurée par observation au microscope électronique à balayage (MEB), en effectuant un ensemble de clichés à un grossissement d'au moins 5000. On mesure ensuite le diamètre d'au moins 200 cristaux à l'aide d'un logiciel d'analyse d'image dédié. La précision est de l'ordre de 3%.

Les cristaux mis en oeuvre dans le cadre de la présente invention présentent de préférence un diamètre moyen en nombre inférieur ou égal à 1,5 $\mu$m, de préférence strictement inférieur à 1,2 $\mu$m, et mieux encore inférieur ou égal à 1,1 $\mu$m.

Comme indiqué précédemment, on préfère utiliser des cristaux de zéolithe de structure faujasite de rapport atomique Si/Al compris entre 1,00 et 1,50, de préférence entre 1,05 et 1,50, et de manière encore plus préférée compris entre 1,10 et 1,50, bornes incluses mesuré par analyse chimique par fluorescence de rayons X.

Lors de l'étape a), outre les cristaux de zéolithe(s) et l'argile, un ou plusieurs additifs peuvent également être ajoutés, par exemple des additifs tels que de la silice, notamment dans le cas où la zéolithe mise en oeuvre est une zéolithe X. La source éventuelle de silice peut être de tout type connu de l'homme du métier, spécialiste de la synthèse de zéolithes, par exemple de la silice colloïdale, des diatomées, de la perlite, des cendres de calcination (« fly ash » en langue anglaise), du sable, ou toute autre forme de silice solide.

Les cristaux de zéolithe mis en oeuvre à l'étape a) peuvent ainsi être avantageusement issus de la synthèse de cristaux de zéolithe X comprenant majoritairement, voir exclusivement des cations sodium, comme c'est le cas par exemple pour les zéolithes NaX (ou 13X), mais on ne sortirait pas du cadre de l'invention en utilisant des cristaux ayant subi un ou plusieurs échanges cationiques, entre la synthèse sous forme NaX et sa mise en oeuvre à l'étape a). Dans ce cas, l'étape d) d'échange cationique devient par conséquent non nécessaire.

Le liant argileux mis en oeuvre à l'étape a) contient au moins 80% de préférence, au moins 90%, de préférence encore au moins 95%, plus particulièrement au moins 96%, en poids, d'argile ou de mélange d'argiles parmi les kaolins, kaolinites, nacrites, dickites, halloysite et/ou métakaolins et peut également contenir d'autres liants minéraux tels que bentonite, attapulgite, sépiolite et autres.

Les proportions d'argiles et de zéolithe(s) mises en oeuvre sont typiquement celles de l'art antérieur, c'est-à-dire de 5 parties à 20 parties en poids d'argile(s) pour 95 parties à 80 parties en poids de zéolithe(s).

Dans tous les cas, les argiles peuvent être utilisées dans leur état brut ou peuvent être préalablement soumises à un ou plusieurs traitements, par exemple choisis parmi calcination, traitement à l'acide, modification chimique, et autres.

Selon un mode de réalisation, lorsque l'argile utilisée est le kaolin, on opère avantageusement, avant l'étape b), une cuisson du mélange obtenu à l'étape a) à une température en général comprise entre 500°C et 600°C afin de convertir le kaolin en méta-kaolin. Le principe en est exposé dans « Zeolite Molecular Sieves » de D.W. Breck, John Wiley and Sons, New York, (1973), pp. 314-315.

L'étape b) de mise en contact avec une solution basique alcaline est typiquement une étape réalisée par immersion du mélange obtenu à l'étape a) dans une solution basique alcaline, en général aqueuse, par exemple une solution aqueuse d'hydroxyde de sodium et/ou d'hydroxyde de potassium. En règle générale, la concentration de la solution alcaline de l'étape b) est comprise entre 0,5 M et 5 M.

Cette étape b) s'opère à froid ou à chaud, de préférence à chaud, à une température supérieure à la température ambiante, et typiquement entre la température ambiante (soit environ 20°C) et la température d'ébullition de la solution alcaline.

Selon une caractéristique du procédé de l'invention, cette immersion est réalisée dans un conteneur clos et étanche, ceci afin d'opérer l'étape c) sous pression, et typiquement sous pression autogène. En variante, il peut être envisagé un moyen externe de mise sous pression, et dans ce cas la pression sera comprise entre au moins la pression autogène et 20 bars.

L'activation qui suit le séchage, est conduite de manière classique, selon les méthodes connues de l'homme du métier, par exemple à une température en général comprise entre 100°C et 400°C, de préférence entre 200°C et 300°C. Cette étape f) d'activation a pour but de fixer la teneur en eau, ainsi que la perte au feu du matériau granulaire de façon optimale pour l'utilisation envisagée. On procède en général par activation thermique qu'on exécute préférentiellement entre 200°C et 300°C pendant une durée déterminée en fonction de la teneur en eau et de la perte au feu souhaitées, typiquement de 1 à 6 heures.

Le procédé de l'invention comprend, lors de l'étape e), éventuellement au moins une étape de mise en forme des matériaux granulaires zéolithiques à la taille souhaitée qui peut être réalisée selon toutes les techniques connues de l'homme du métier, telles que par exemple assiette granulatrice, broyage, concassage, et autres, de préférence concassage.

Le procédé de l'invention comprend également une ou plusieurs étapes optionnelles de mise en forme qui peu(ven)t être réalisée(s) selon toutes les techniques connues de l'homme du métier, telles que par exemple extrusion, compactage, agglomération, et autres. Ces étapes de mise en forme peuvent être effectuées après l'une ou l'autre ou plusieurs des étapes a), b), c), d), e) ou f). On préfère effectuer une seule étape de mise en forme après l'étape a), b), c), d), e) ou f), et de préférence après l'étape a), b), ou c).

L'opération de mise en forme peut éventuellement faire appel à un liant d'agglomération. Selon un mode de réalisation préféré de l'invention ledit liant comprend au moins 80% d'une argile ou d'un mélange d'argiles, éventuellement zéolithisable(s), et avec jusqu'à 5% d'additifs ainsi qu'avec la quantité d'eau permettant la mise en forme du matériau aggloméré. Le matériau granulaire zéolithique peut ainsi être mis sous forme de bille, d'extrudé ou autre, de taille comprise entre 0,1 mm et 1 mm, comme indiqué précédemment.

Le matériau granulaire zéolithique selon l'invention est particulièrement adapté pour les procédés de séparation de composés en phase liquide, et notamment pour les procédés dans lesquels ledit matériau est soumis à des contraintes mécaniques importantes, par exemple les procédés de séparation en phase liquide à co-courant ou à contre-courant, et plus particulièrement les procédés de séparation en phase liquide en lit mobile simulé. Le matériau granulaire zéolithique selon l'invention est tout particulièrement adapté pour les procédés de séparation des xylènes en phase liquide.

Ainsi, et selon encore un autre aspect, la présente invention concerne l'utilisation d'au moins un matériau granulaire zéolithique tel qu'il vient d'être défini, comme matériau adsorbant dans les procédés de séparation en phase liquide à co-courant ou à contre-courant, et plus particulièrement dans les procédés de séparation en phase liquide en lit mobile simulé, typiquement dans les procédés de séparation des coupes aromatiques comportant des mélanges d'isomères aromatiques à 8 atomes de carbone et plus particulièrement dans les procédés en phase liquide de séparation des xylènes en lit mobile simulé, et tout particulièrement dans les procédés de récupération de para-xylène de haute pureté à partir de coupes d'isomères aromatiques à 8 atomes de carbone.

Enfin, l'invention concerne également le procédé de séparation des coupes aromatiques comportant des mélanges d'isomères à 8 atomes de carbone et plus particulièrement le procédé en phase liquide de séparation des xylènes en lit mobile simulé, et tout particulièrement le procédé de récupération de para-xylène de haute pureté à partir de coupes d'isomères aromatiques à 8 atomes de carbone, comme par exemple décrit dans la demande WO2009/081024, et dans lequel est mis en oeuvre au moins un matériau granulaire zéolithique tel que décrit précédemment.

Les exemples suivants permettent d'illustrer l'objet de l'invention, et sont fournis à titre indicatif seulement, sans toutefois être destinés en aucune façon à limiter les divers modes de réalisation de la présente invention.

La quantité des fractions zéolithiques dans le matériau granulaire zéolithique est mesurée par analyse par diffraction de rayons X, connue de l'homme du métier sous l'acronyme DRX. Cette analyse est réalisée sur un appareil de la marque Bruker, puis la quantité des fractions zéolithiques est évaluée au moyen du logiciel TOPAS de la société Bruker.

Le matériau granulaire zéolithique a été évalué quant au rapport atomique Si/Al et au taux d'échange cationique par analyse chimique élémentaire du matériau granulaire zéolithique, et plus précisément par analyse chimique par fluorescence de rayons X telle que décrite dans la norme NF EN ISO 12677 : 2011 sur un spectromètre dispersif en longueur d'onde (WDXRF), par exemple Tiger S8 de la société Bruker. Le spectre de fluorescence X a l'avantage de dépendre très peu de la combinaison chimique de l'élément, ce qui offre une détermination précise, à la fois quantitative et qualitative. On obtient de manière classique après étalonnage pour chaque oxyde $SiO_2$ et $Al_2O_3$, ainsi que oxydes de sodium, potassium et baryum, une incertitude de mesure inférieure à 0,4% en poids. L'incertitude de mesure du rapport atomique Si/Al est de $\pm$ 5%.

La détermination de la taille du matériau granulaire est effectuée par analyse de la distribution granulométrique d'un échantillon d'aggloméré par imagerie selon la norme ISO 13322-2:2006, en utilisant un tapis roulant permettant à l'échantillon de passer devant l'objectif de la caméra. La taille de l'objet (diamètre moyen en nombre) est ensuite calculée à partir de la distribution granulométrique en appliquant la norme ISO 9276-2:2001. La précision est de l'ordre de 0,01 mm pour la gamme de taille du matériau granulaire zéolithique de l'invention.

Les volumes macroporeux et mésoporeux sont mesurés par porosimétrie par intrusion de mercure. Un porosimètre à mercure type Autopore® 9500 de Micromeritics est utilisé pour analyser la répartition du volume poreux contenu dans les macropores et dans les mésopores.

La méthode expérimentale, décrite dans le manuel opératoire de l'appareil faisant référence à la norme ASTM D4284-83, consiste à placer un échantillon d'adsorbant (matériau granulaire zéolithique à mesurer) (de perte au feu connue) préalablement pesé, dans une cellule du porosimètre, puis, après un dégazage préalable (pression d'évacuation de 30 $\mu$m Hg pendant au moins 10 min), à remplir la cellule avec du mercure à une pression donnée (0,0036 MPa), et ensuite à appliquer une pression croissante par palier jusqu'à 400 MPa afin de faire pénétrer progressivement le mercure dans le réseau poreux de l'échantillon.

La relation entre la pression appliquée et le diamètre apparent des pores est établie en supposant des pores cylindriques, un angle de contact entre le mercure et la paroi des pores de 140° et une tension de surface du mercure de 485 dynes/cm. La quantité cumulée de mercure introduite en fonction de la pression appliquée est enregistrée. On fixe à 0,2 MPa la valeur à partir de laquelle le mercure remplit tous les vides inter-granulaires, et on considère qu'au delà le mercure pénètre dans les pores du matériau granulaire. Le volume de grain (Vg) est alors calculé en soustrayant le volume cumulé de mercure à cette pression (0,2 MPa) au volume de la cellule du porosimètre, et en divisant cette différence par la masse du matériau granulaire équivalent anhydre, c'est-à-dire la masse dudit matériau corrigée de la perte au feu.

Le volume macroporeux du matériau granulaire est défini comme étant le volume cumulé de mercure introduit à une pression comprise entre 0,2 MPa et 30 MPa, correspondant au volume contenu dans les pores de diamètre apparent supérieur à 50 nm. Le volume mésoporeux du matériau granulaire est défini comme étant le volume cumulé de mercure introduit à une pression comprise entre 30 MPa et 400 MPa.

Dans le présent document, les volumes macroporeux et mésoporeux des adsorbants zéolithiques, exprimés en $cm^3 \cdot g^{-1}$, sont ainsi mesurés par intrusion de mercure et rapportés à la masse de l'échantillon en équivalent anhydre, c'est-à-dire la masse dudit matériau corrigée de la perte au feu.

Le volume microporeux est déterminé selon toutes méthodes connues de l'homme du métier, par exemple à partir de la mesure de l'isotherme d'adsorption d'un gaz à sa température de liquéfaction, par exemple azote, argon, oxygène, et autres. Préalablement à cette mesure d'adsorption, le matériau granulaire zéolithique de l'invention est dégazé entre 300°C et 450°C pendant une durée de 9 heures à 16 heures, sous vide (P < 6,7.10$^{-4}$ Pa). Par exemple, pour une zéolithe de structure FAU, la mesure de l'isotherme d'adsorption d'azote à 77K est ensuite effectuée sur un appareil de type ASAP 2010 M de Micromeritics, en prenant au moins 35 points de mesure à des pressions relatives de rapport PIPa compris entre 0,002 et 1. Le volume microporeux est déterminé selon l'équation de Dubinin et Raduskevitch à partir de l'isotherme obtenu, en appliquant la norme ISO 15901-3:2007. Le volume microporeux ainsi évalué s'exprime en cm$^3$ d'adsorbat liquide par gramme d'adsorbant. L'incertitude de mesure est de $\pm$ 0,003 g/cm$^3$.

La résistance mécanique mesurée est la résistance à l'écrasement en lit (REL) caractérisée selon la méthode Shell série SMS1471-74 (Shell Method Series SMS1471-74 « Détermination of Bulk Crushing Strength of Catalysts. Compression-Sieve Method »), associée à l'appareil « BCS Tester » commercialisé par la société Vinci Technologies.

Cette méthode de mesure de la REL, initialement destinée à la caractérisation de catalyseurs de 3 mm à 6 mm est basée sur l'utilisation d'un tamis de 425 $\mu$m qui va permettre notamment de séparer les fines créées lors de l'écrasement. L'utilisation d'un tamis de 425 $\mu$m reste adaptée pour des particules de diamètre supérieur à 1,6 mm mais doit être adaptée selon la granulométrie du matériau que l'on cherche à caractériser. Pour le matériau granulaire zéolithique de la présente invention, un tamis de 200 $\mu$m est utilisé à la place du tamis de 425 $\mu$m mentionné dans la méthode Shell standard SMS1471-74.

Le protocole de mesure est le suivant : un échantillon de 20 cm$^3$ de matériau à analyser, préalablement tamisé avec le tamis adapté (200 $\mu$m) et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 cm$^3$ de billes d'acier afin de mieux répartir la force exercée par le piston sur le matériau (utilisation de billes de 2 mm de diamètre pour des particules de forme sphérique de diamètre strictement inférieur à 1,6 mm). Les fines obtenues aux différents paliers de pression sont séparées par tamisage (tamis adapté de 200 $\mu$m) et pesées.

La REL est déterminée par la pression en mégaPascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit de matériau zéolithique et en interpolant à 0,5 % massique de fines cumulées. La résistance mécanique à l'écrasement en lit est typiquement comprise entre quelques centaines de kPa et quelques dizaines de MPa et généralement comprise entre 0,3 MPa et 3,2 MPa. La précision est de manière classique inférieure à 0,1 MPa.

Les Figures annexées sont des clichés obtenus lors de l'observation par microscopie électronique à balayage (MEB) d'une section polie de différents matériaux granulaires zéolithiques. La surface polie du matériau a été préparée par polissage ionique (ion Argon). L'appareil utilisé pour la préparation est un appareil Ilion+™ commercialisé par Gatan, avec les paramètres suivants : tension d'accélération de 5 kV, durée de polissage de 4 heures, angle de tilt 0°, température ambiante, mode cross-section.

Les Figures annexées sont commentées ci-dessous :

- Figure 1a : Matériau granulaire zéolithique non connexe correspondant à l'Exemple 1, grossissement 5000 ;
- Figure 1b: Matériau granulaire zéolithique non connexe correspondant à l'Exemple 1, grossissement 10000 ;
- Figure 2a : Matériau granulaire zéolithique connexe correspondant à l'Exemple 2, grossissement 5000 ;
- Figure 2b : Matériau granulaire zéolithique connexe correspondant à l'Exemple 2, grossissement 10000.

Pour l'observation de l'échantillon, un microscope électronique à balayage à canon à émission de champ (MEB FEG) est utilisé dans les conditions suivantes : tension d'accélération de 2 kV, imagerie avec un détecteur d'électrons secondaires latéral de type Everhart-Thornley, référence de grandissement Polaroid 545. L'échantillon n'a pas subi de métallisation.

Les exemples suivants permettent d'illustrer l'objet de l'invention. Ils sont fournis à titre indicatif seulement, et ne sont destinés en aucune façon à limiter les divers modes de réalisation de la présente invention.

**Exemple 1 (comparatif)** :

**Préparation d'un matériau non connexe à base de NaX**

On prépare un adsorbant zéolithique à base de poudre de zéolithe X de taille de cristaux 1 $\mu$m de rapport atomique Si/Al = 1,25, obtenu en mélangeant intimement et en agglomérant 100 g (exprimés en équivalent calciné) de poudre de zéolite NaX de taille de cristaux 1 $\mu$m et de rapport atomique Si/Al = 1,25 avec 15 g de kaolin, (exprimés en équivalent calciné) et 7,5 g de silice colloïdale vendue sous la dénomination commerciale Klebosol®30 (contenant 30 % en poids de SiO$_2$ et 0,5% de Na$_2$O) avec la quantité d'eau adéquate pour opérer par extrusion.

Les adsorbants sont séchés, concassés de manière à récupérer des grains dont le diamètre équivalent moyen en nombre est égal à 0,5 mm, puis calcinés à 550°C sous courant d'azote pendant 2 heures.

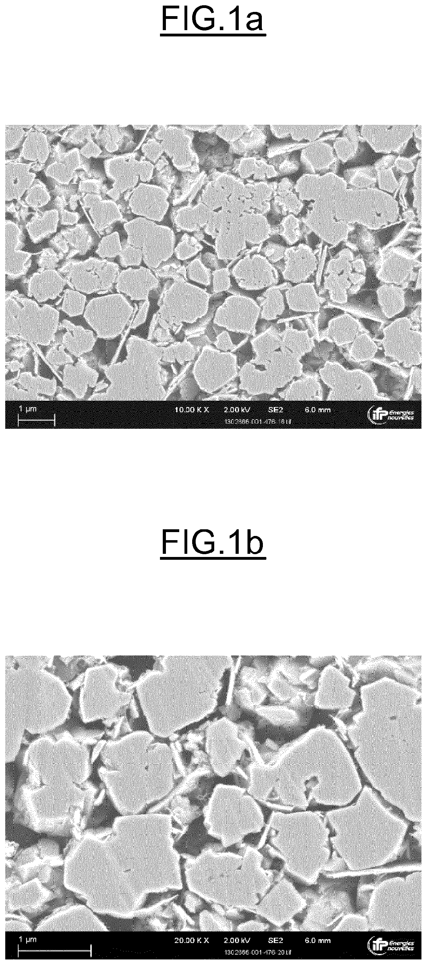

À cette étape, on réalise une observation d'une section polie du matériau selon l'invention par MEB : sur 10 clichés avec un grossissement de 10000, on observe un agglomérat de cristaux de zéolithes, tel que les cristaux ne forment pas un ensemble connexe sur tout le volume dudit matériau, la surface connexe représentant environ 5% de la surface occupée par le matériau sur le cliché (Figure 1a).

La résistance mécanique en lit REL de ces agglomérés mesurée telle que décrit ci-dessus est de 0,8 MPa. La proportion des mésopores en volume par rapport au volume total macroporeux et mésoporeux est de 0,13 (

$$\frac{Vme}{Vme + Vma} = 0,13$$

), la proportion de volume microporeux rapporté à la somme des volumes macroporeux,

$$\frac{Vmi}{Vma + Vme + Vmi} = 0,60$$

mésoporeux et microporeux est de 0,60 (), et la proportion des volumes mésopo-

$$\frac{Vma + Vme}{Vg} = 0,39$$

reux et macroporeux rapportée au volume total du matériau granulaire est de 0,39 ().

## Exemple 2 (selon l'invention) :

### Synthèse d'un matériau zéolithique connexe à base de NaX

On utilise un adsorbant zéolithique tel que réalisé à l'exemple 1.

Ces extrudés séchés et calcinés sont mis en contact avec une solution basique préparée en mélangeant 200 g d'hydroxyde de sodium (NaOH à 50% en poids dans l'eau) avec 300 g d'eau sous agitation douce. Le mélange est ensuite transvasé dans des tubes en matière plastique qui sont par la suite scellés. Le système est maintenu à une température régulée à 120°C pendant 2 heures sous pression autogène.

Les adsorbants sont récupérés puis lavés à l'eau jusqu'à ce que le pH soit proche de 10. Les extrudés sont séchés, concassés de manière à récupérer des grains dont le diamètre équivalent moyen en nombre est égal à 0,5 mm, puis calcinés à 550°C sous courant d'azote pendant 2 heures.

L'adsorbant à cette étape est observé par MEB sur sections polies préparées par polissage ionique. L'observation de 10 clichés pris sur différentes sections polies, avec un grossissement de 10000 montre une porosité et un matériau homogène. L'analyse des clichés MEB montre, en moyenne sur les 10 clichés, une surface connexe représentant environ 78% de la surface occupée par le matériau sur chaque cliché, ce qui correspond à un matériau connexe au sens de l'invention.

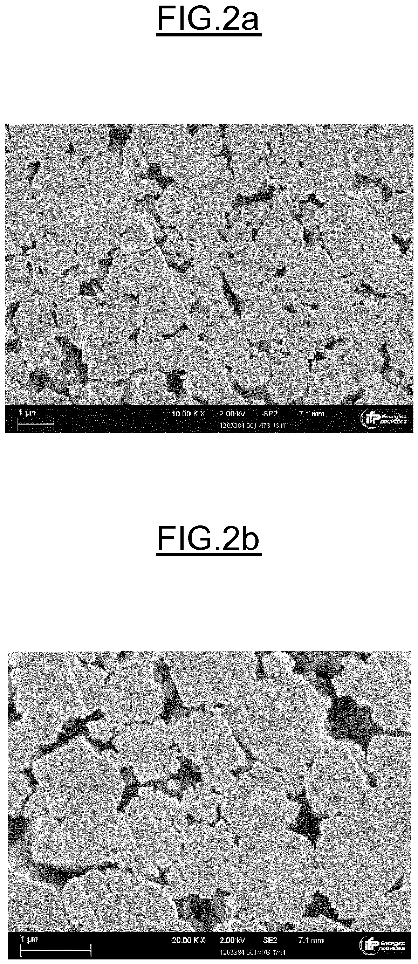

Le renforcement de la résistance mécanique est d'ailleurs observé lors du polissage dans les conditions décrites, par un effet rideau (strie observée sur la Figure 2a ou la Figure 2b), caractéristique d'un matériau dur. Cet effet est absent lors du polissage du matériau représenté sur la Figure 1a ou la Figure 1b, qui est un agglomérat de cristaux de zéolithes, tel que les cristaux ne forment pas un ensemble connexe.

La résistance mécanique en lit REL de ces agglomérés mesurée telle que décrit ci-dessus est de 2,5 MPa. La proportion des mésopores en volume par rapport au volume total macroporeux et mésoporeux est de 0,05 (

$$\frac{Vme}{Vme + Vma} = 0,05$$

), la proportion de volume microporeux rapporté à la somme des volumes macroporeux,

$$\frac{Vmi}{Vma + Vme + Vmi} = 0,52$$

mésoporeux et microporeux est de 0,52 (), et la proportion des volumes mésoporeux

$$\frac{Vma + Vme}{Vg} = 0,32$$

et macroporeux rapportée au volume total du matériau granulaire est de 0,32 ().

**Exemple 3 (selon l'invention) :**

**Synthèse d'un matériau zéolithique connexe à base de BaX**

Dans un récipient, on mélange 50 g d'hydroxyde de sodium à 50% en poids dans l'eau, 20 g (exprimés en équivalent anhydre) de poudre de zéolithe NaX de taille de cristaux de 0,8 $\mu$m et de rapport atomique Si/Al = 1,20, 1 g de kaolin (exprimés en équivalent calciné) préalablement calciné à 550°C pendant 2 heures et 0,5 g de silice colloïdale vendue sous la dénomination commerciale Klebosol®30 (contenant 30% en poids de $SiO_2$ et 0,5% de $Na_2O$) avec 25 g d'eau.

Le mélange ainsi obtenu est transvasé dans des tubes en matière plastique qui sont par la suite scellés. Le système est maintenu à une température régulée à 120°C pendant 2 heures sous pression autogène.

Les adsorbants sont récupérés puis lavés à l'eau jusqu'à ce que le pH soit proche de 10. Les extrudés sont séchés, concassés de manière à récupérer des grains dont le diamètre équivalent moyen en nombre est égal à 0,5 mm, puis calcinés à 550°C sous courant d'azote pendant 2 heures.

Ces adsorbants sont soumis à un échange cationique par action d'une solution aqueuse de chlorure de baryum 0,5 M à 95°C en 4 étapes. À chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel. Les agglomérats sont ensuite séchés à 80°C pendant 2 heures et enfin activés à 250°C pendant 2 heures sous courant d'azote.

La résistance mécanique en lit REL de ces agglomérés mesurée telle que décrit ci-dessus est de 2 MPa. La proportion des mésopores en volume par rapport au volume total macroporeux et mésoporeux est de 0,08 (

$$\frac{Vme}{Vme + Vma} = 0{,}08$$

), la proportion de volume microporeux rapporté à la somme des volumes macroporeux,

$$\frac{Vmi}{Vma + Vme + Vmi} = 0{,}53$$

mésoporeux et microporeux est de 0,53 ( ), et la proportion des volumes mésopo-

$$\frac{Vma + Vme}{Vg} = 0{,}33$$

reux et macroporeux rapportée au volume total du matériau granulaire est de 0,33 ( ).

## Revendications

1. Matériau granulaire zéolithique de taille comprise entre 0,1 mm et 1 mm, de préférence comprise entre 0,2 mm et 1 mm, et en particulier comprise entre 0,3 mm et 0,8 mm, plus généralement entre 0,4 mm et 0,6 mm, bornes incluses, dont la structure zéolithique forme un ensemble connexe sur tout le volume dudit matériau, et présentant une résistance mécanique à l'écrasement en lit supérieure ou égale à 1,0 MPa, plus généralement supérieure ou égale à 1,5 MPa, de préférence supérieure ou égale à 2,0 MPa, typiquement supérieure ou égale à 2,1 MPa, un ensemble connexe étant une structure tridimensionnellement connexe par arcs, c'est-à-dire que tout couple de points de la structure zéolithique est relié par un chemin, cet ensemble étant évalué par analyse d'images, sur une moyenne de 10 clichés de différentes sections polies du matériau, lesdits clichés étant obtenus à l'aide d'un microscope électronique à balayage (MEB), avec un grossissement compris entre 5000 et 10000, l'ensemble étant connexe lorsque la proportion de surface connexe représente au moins 50% de la surface occupée par le matériau sur chaque cliché.

2. Matériau granulaire zéolithique selon la revendication 1, comprenant au moins une zéolithe choisie parmi les zéolithes de structure FAU et les zéolithes de structure EMT, LTA ou MFI.

3. Matériau granulaire zéolithique selon la revendication 1 ou la revendication 2, comprenant au moins une zéolithe choisie parmi les zéolithes LSX, MSX et X de préférence la zéolithe X.

4. Matériau granulaire zéolithique selon l'une quelconque des revendications précédentes, comprenant plus de 94% en poids de zéolithe(s), de préférence entre 96% et 98% en poids de zéolithe(s), bornes incluses, par rapport au poids total du matériau granulaire zéolithique.

**5.** Matériau granulaire zéolithique selon l'une quelconque des revendications précédentes, dont la distribution poreuse satisfait aux inéquations 1), 2) et 3) suivantes :

1) $\dfrac{Vme}{Vme + Vma} \leq 0,1,$ de préférence $0,01 \leq \dfrac{Vme}{Vme + Vma} \leq 0,1,$ plus préférentiellement $0,01 \leq \dfrac{Vme}{Vme + Vma} \leq 0,06$ ;

2) $0,4 \leq \dfrac{Vmi}{Vma + Vme + Vmi},$ de préférence $0,4 \leq \dfrac{Vmi}{Vma + Vme + Vmi} \leq 0,6,$ de préférence encore $0,45 \leq \dfrac{Vmi}{Vma + Vme + Vmi} \leq 0,55$ ; et

3) $0,25 \leq \dfrac{Vma + Vme}{Vg} \leq 0,35$ ;

où « Vma » représente le volume macroporeux, « Vme » représente le volume mésoporeux, « Vmi » représente le volume microporeux et « Vg » représente le volume total du matériau granulaire, où les volumes macroporeux et mésoporeux sont mesurés par intrusion de mercure et le volume microporeux est mesuré par adsorption d'azote.

**6.** Procédé de préparation d'un matériau granulaire zéolithique selon l'une quelconque des revendications 1 à 5, comprenant au moins les étapes de :

a) mélange de cristaux d'au moins une zéolithe, avec un liant argileux contenant au moins 80%, de préférence au moins 90%, de préférence encore au moins 95% en poids d'argile zéolithisable, et éventuellement une source de silice,
b) mise en contact avec une solution basique alcaline, typiquement une solution d'hydroxyde de sodium ou d'un mélange d'hydroxyde de sodium et d'hydroxyde de potassium,
c) mise en température de la suspension entre 80°C et 600°C, de préférence entre 80°C et 150°C, dans un conteneur clos et étanche, sous une pression au moins égale à la pression autogène, typiquement comprise entre la pression autogène et 20 bars,
d) échange cationique éventuel des cations contenu dans le milieu réactionnel de l'étape c) par mise en contact avec une solution d'ions baryum, ou d'ions baryum et d'ions potassium,
e) lavage et séchage des matériaux granulaires zéolithiques ainsi obtenus, et éventuellement au moins une étape de mise en forme à la taille souhaitée, et
f) activation par chauffage à une température en général comprise entre 100°C et 400°C, de préférence entre 200°C et 300°C du matériau granulaire zéolithique obtenus à l'étape e).

**7.** Procédé selon la revendication 6, dans lequel le liant argileux mis en oeuvre à l'étape a) contient au moins 80% de préférence, au moins 90%, de préférence encore au moins 95%, plus particulièrement au moins 96%, en poids, d'argile ou de mélange d'argiles parmi les kaolins, kaolinites, nacrites, dickites, halloysite et/ou métakaolins et peut également contenir d'autres liants minéraux tels que bentonite, attapulgite, sépiolite et autres.

**8.** Utilisation d'au moins un matériau granulaire zéolithique selon l'une quelconque des revendications 1 à 5, comme matériau adsorbant dans les procédés de séparation en phase liquide à co-courant ou à contre-courant, et plus particulièrement dans les procédés de séparation en phase liquide en lit mobile simulé, typiquement dans les procédés de séparation des coupes aromatiques comportant des mélanges d'isomères aromatiques à 8 atomes de carbone et plus particulièrement dans les procédés en phase liquide de séparation des xylènes en lit mobile simulé, et tout particulièrement dans les procédés de récupération de para-xylène de haute pureté à partir de coupes d'isomères aromatiques à 8 atomes de carbone.

**9.** Procédé de séparation des coupes aromatiques comportant des mélanges d'isomères à 8 atomes de carbone, dans lequel est mis en oeuvre au moins un matériau granulaire zéolithique selon l'une quelconque des revendications 1 à 5.

**10.** Procédé selon la revendication 9, qui est un procédé en phase liquide de séparation des xylènes en lit mobile simulé, et tout particulièrement un procédé de récupération de para-xylène de haute pureté à partir de coupes d'isomères aromatiques à 8 atomes de carbone.

**Patentansprüche**

**1.** Körniges Zeolithmaterial mit einer Größe im Bereich zwischen 0,1 mm und 1 mm, vorzugsweise im Bereich zwischen 0,2 mm und 1 mm und insbesondere im Bereich zwischen 0,3 mm und 0,8 mm, allgemeiner zwischen 0,4 mm und 0,6 mm, darin eingeschlossen die Ränder, wobei die Zeolithstruktur eine verbundene Einheit auf dem gesamten Volumen des Materials bildet und einen mechanischen Widerstand gegen die Quetschung im Bett von mehr als oder gleich wie 1,0 MPa, allgemeiner von mehr als oder gleich wie 1,5 MPa, vorzugsweise von mehr als oder gleich wie 2,0 MPa, typischerweise von mehr als oder gleich wie 2,1 MPa aufweist,
wobei eine verbundene Einheit eine dreidimensionale Struktur ist, die durch Bögen verbunden ist, d. h. dass jedes Punktpaar der Zeolithstruktur durch einen Weg verbunden ist, wobei diese Einheit durch Bildanalyse auf durchschnittlich 10 Negativen von verschiedenen polierten Abschnitten des Materials bewertet wird, wobei die Negative mit Hilfe eines Rasterelektronenmikroskops (MEB) mit einer Vergrößerung im Bereich zwischen 5000 und 10000 erhalten werden, wobei die Einheit verbunden ist, wenn der verbundene Oberflächenteil mindestens 50 % der Oberfläche darstellt, der auf jedem Negativ vom Material bedeckt ist.

**2.** Körniges Zeolithmaterial nach Anspruch 1, umfassend mindestens einen Zeolith, ausgewählt aus den Zeolithen mit FAU-Struktur und den Zeolithen mit EMT-, LTA- oder MFI-Struktur.

**3.** Körniges Zeolithmaterial nach Anspruch 1 oder Anspruch 2, umfassend mindestens einen Zeolith, ausgewählt aus den LSX-, MSX- und X-Zeolithen, vorzugsweise dem X-Zeolith.

**4.** Körniges Zeolithmaterial nach einem der vorhergehenden Ansprüche, umfassend mehr als 94 Gew% Zeolith(en), vorzugsweise zwischen 96 Gew% und 98 Gew% Zeolith(en), darin eingeschlossen die Ränder, mit Bezug auf das Gesamtgewicht des körnigen Zeolithmaterials.

**5.** Körniges Zeolithmaterial nach einem der vorhergehenden Ansprüche, wobei die poröse Verteilung den Folgenden Ungleichungen 1), 2) und 3) entspricht

$$1) \quad \frac{Vme}{Vme + Vma} \leq 0,1, \quad \text{vorzugsweise} \quad 0,01 \leq \frac{Vme}{Vme + Vma} \leq 0,1, \quad \text{bevorzugter}$$

$$0,01 \leq \frac{Vme}{Vme + Vma} \leq 0,06 \; ;$$

$$2) \quad 0,4 \leq \frac{Vmi}{Vma + Vme + Vmi}, \quad \text{vorzugsweise} \quad 0,4 \leq \frac{Vmi}{Vma + Vme + Vmi} \leq 0,6, \quad \text{noch}$$

$$\text{bevorzugter} \quad 0,45 \leq \frac{Vmi}{Vma + Vme + Vmi} \leq 0,55 \; ; \quad \text{und}$$

$$3) \quad 0,25 \leq \frac{Vma + Vme}{Vg} \leq 0,35 \; ;$$

wobei "Vma" das makroporöse Volumen darstellt, "Vme" das mesoporöse Volumen darstellt, "Vmi" das mikroporöse Volumen darstellt und "Vg" das Gesamtvolumen des körnigen Materials darstellt, wobei das makroporöse und das mesoporöse Volumen durch Eindringen von Quecksilber gemessen werden und das mikroporöse Volumen durch Adsorption von Stickstoff gemessen wird.

**6.** Verfahren zur Herstellung eines körnigen Zeolithmaterials nach einem der Ansprüche 1 bis 5, umfassend mindestens die folgenden Schritte:

a) Mischen von Kristallen mindestens eines Zeoliths mit einem Tonbinder, enthaltend mindestens 80 Gew%, vorzugsweise mindestens 90 Gew%, noch bevorzugter mindestens 95 Gew% zeolithisierbaren Tons und eventuell eine Siliciumquelle,

b) In-Kontakt-Bringen mit einer alkalinen basischen Lösung, typischerweise einer Lösung von Natriumhydroxyd oder eine Mischung von Natriumhydroxyd und Kaliumhydroxyd,

c) Erhitzen der Suspension auf zwischen 80 ºC und 600 ºC, vorzugsweise zwischen 80 °C und 150 °C, in einem geschlossenen und dichten Behälter, unter einem Druck, der mindestens gleich dem autogenen Druck ist, typischerweise im Bereich zwischen dem autogenen Druck und 20 bar,

d) eventuelles Kationenaustauschen der Kationen, die in dem Reaktionsmedium von Schritt c) enthalten sind, durch In-Kontakt-Bringen einer Lösung von Baryumionen oder Baryumionen mit Kaliumionen,

e) Waschen und Trocknen der so erhaltenen körnigen Zeolithmaterialien und eventuell mindestens einen Schritt des Formens in die gewünschte Größe, und

f) Aktivieren durch Erhitzen auf eine Temperatur, die im Allgemeinen im Bereich zwischen 100 °C und 400 °C, vorzugsweise zwischen 200 °C und 300 °C liegt, des körnigen Zeolithmaterials, erhalten in Schritt e).

7.  Verfahren nach Anspruch 6, wobei der Tonbinder, eingesetzt in Schritt a), mindestens 80 Gew%, vorzugsweise mindestens 90 Gew%, noch bevorzugter mindestens 95 Gew%, insbesondere mindestens 96 Gew% Ton oder eine Mischung von Tonarten aus Kaolinen, Kaoliniten, Nakriten, Dickiten, Halloysite und/oder Metakaolinen enthält und auch andere anorganische Bindemittel enthalten kann, wie z. B. Betonit, Attapulgit, Sepiolith und andere.

8.  Verwendung mindestens eines körnigen Zeolithmaterials nach einem der Ansprüche 1 bis 5 als adsorbierendes Material bei den Verfahren zur Trennung in flüssige Phase im Gleichstrom oder im Gegenstrom und insbesondere bei den Verfahren zur Trennung in flüssiger Phase in simuliertem beweglichem Bett, typischerweise bei den Verfahren zur Trennung der aromatischen Fraktionen, umfassend Mischungen von aromatischen Isomeren mit 8 Kohlenstoffatomen und insbesondere bei den Verfahren in der flüssigen Phase zur Trennung von Xylenen in simuliertem beweglichem Bett, und ganz besonders bei den Verfahren zur Wiedergewinnung von hochreinem Paraxylen ausgehend von aromatischen Isomerfraktionen mit 8 Kohlenstoffatomen.

9.  Verfahren zur Trennung der aromatischen Fraktionen, umfassend Mischungen von Isomeren mit 8 Kohlenstoffatomen, wobei mindestens ein körniges Zeolithmaterial nach einem der Ansprüche 1 bis 5 eingesetzt wird.

10. Verfahren nach Anspruch 9, wobei es sich um ein Verfahren in flüssiger Phase zur Trennung von Xylenen in simuliertem beweglichem Bett und ganz besonders ein Verfahren zur Wiedergewinnung von hochreinem Paraxylen ausgehend von aromatischen Isomerfraktionen mit 8 Kohlenstoffatomen handelt.

**Claims**

1.  A zeolitic granular material having a particle size of from 0.1 mm to 1 mm, preferably from 0.2 mm to 1 mm, and particularly from 0.3 mm to 0.8 mm, more generally from 0.4 to 0.6 mm, limits inclusive, wherein the zeolitic structure forms an assembly connected over the entire volume of said material, and presenting a mechanical bulk crushing strength of greater than or equal to 1.0 MPa, more generally greater than or equal to 1.5 MPa, preferably greater than or equal to 2.0 MPa, typically greater than or equal to 2.1 MPa,
    an assembly connected being a three-dimensionally connected by arcs, *i.e.* any pair of points in the zeolite structure is connected via a path, this assembly being evaluated by image analysis, for an average of ten images on the various polished sections of the material, said images being obtained with a scanning electron microscope (SEM), with a magnification of between 5000 and 10000, an assembly being connected when the proportion of connected surface represents at least 50% of the surface occupied by the material on each image.

2.  The zeolitic granular material of claim 1, comprising at least one zeolite chosen among zeolites of structure FAU and zeolites of structure EMT, LTA or MFI.

3.  The zeolitic granular material of claim 1 or claim 2, comprising at least one zeolite chosen among zeolites LSX, MSX and X, preferably zeolite X.

4.  The zeolitic granular material according to any of preceding claims, comprising greater than 94% by weight of zeolite(s), preferably between 96% and 98% by weight of zeolite(s), limits inclusive, relative to the total weight of the zeolitic granular material.

5. The zeolitic granular material according to any of preceding claims, wherein the pore distribution satisfies the inequalities 1), 2) and 3) below:

$$1)\ \frac{V_{me}}{V_{me}+V_{ma}} \leq 0.1; \text{ preferably } 0.01 \leq \frac{V_{me}}{V_{me}+V_{ma}} \leq 0.1,$$

$$\text{more preferably } 0.01 \leq \frac{V_{me}}{V_{me}+V_{ma}} \leq 0.06\ ;$$

$$2)\ 0.4 \leq \frac{V_{mi}}{V_{me}+V_{ma}+V_{mi}}\ ; \text{ preferably } 0.4 \leq \frac{V_{mi}}{V_{me}+V_{ma}+V_{mi}} \leq 0.6\ ;$$

$$\text{more preferably } 0.45 \leq \frac{V_{mi}}{V_{me}+V_{ma}+V_{mi}} \leq 0.55\ ;$$

and

$$3)\ 0.25 \leq \frac{V_{ma}+V_{me}}{V_g} \leq 0.35\ ;$$

wherein "Vma" represents the macropore volume, "Vme" represents the mesopore volume, "Vmi" represents the micropore volume, and "Vg" represents the total volume of the granular material, in which the macropore and mesopore volumes are measured by mercury intrusion and the micropore volume is measured by nitrogen adsorption.

6. A process for preparing the zeolitic granular material according to any of preceding claims 1 to 5, comprising at least the following steps:

a) mixing crystals of at least one zeolite with a clay binder containing at least 80% by weight, preferably at least 90% by weight, more preferably at least 95 % by weight of zeolitizable clay, and, optionally, a source of silica,
b) placing the mixture in contact with an alkaline basic solution, typically a sodium hydroxide solution or a mixture of sodium hydroxide and potassium hydroxide,
c) bringing the suspension to a temperature between 80° C. and 600° C., preferably between 80°C and 150°C, in a closed and leak tight container, at a pressure at least equal to the autogenous pressure, typically comprised between the autogenous pressure and 20 bars,
d) optionally, carrying out cationic exchange of the cations contained in the reaction medium of step c) by placing the reaction medium in contact with a solution of barium ions, or of barium ions and potassium ions,
e) washing and drying of the zeolitic granular material thus obtained, and, optionally, at least one step of forming the zeolitic granular material to a desired size, and
f) activating the zeolitic granular material obtained in e) by heating to a temperature between 100° C and 400° C, preferably between 200 and 300°C.

7. The process of claim 6, wherein the clay binder prepared at step a) contains at least 80% by weight, preferably at least 90% by weight, more preferably 95% by weight, more particularly at least 96% by weight, of clay or of mixtures of clays from among kaolins, kaolinites, nacrites, dickites, halloysite and/or metakaolins, and may also contain other mineral binders such as bentonite, attapulgite, sepiolite, and the like.

8. The use of at least on granular zeolitic material according to any of preceding claims 1 to 5, as an adsorbent material in co-current or counter-current liquid-phase separation processes, and particularly simulated mobile bed liquid-phase separation processes, typically in processes for separating aromatic fractions comprising mixtures of isomers containing 8 carbon atoms, and more particularly in liquid-phase processes for separating xylenes in a simulated mobile bed, and most particularly in processes for recovering high-purity paraxylene from aromatic isomer fractions containing 8 carbon atoms.

9. A process for separating aromatic fractions comprising mixtures of isomers containing 8 carbon atoms, wherein contacting an aromatic fraction comprising mixtures of isomers containing 8 carbon atoms with the zeolitic granular material according to any of preceding claims 1 to 5.

10. The process of claim 9, wherein the process is a liquid-phase process for separating xylenes in a simulated mobile bed, and particularly in processes for recovering high-purity para-xylene from aromatic isomer fractions containing 8 carbon atoms.

# FIG.1a

# FIG.1b

# FIG.2a

# FIG.2b

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20120093715 A
- US 20130052126 A
- WO 2000000287 A
- WO 2008009845 A
- WO 2009081023 A
- WO 2009081024 A

**Littérature non-brevet citée dans la description**

- Principles of Adsorption and Adsorption Processes » (« Principes de l'Adsorption et des Procédés d'Adsorption. John Wiley & Sons, 1984, 326, , 407
- **QIU.** *Micropor. Mesopor. Mater.,* 1998, vol. 21 (4-6), 245-251
- **AKHTAR ; BERGSTRÖM.** *J. Am. Chem. Soc.,* 2001, vol. 94 (1), 92-98
- **D.W. BRECK.** Zeolite Molecular Sieves. John Wiley and Sons, 1973, 314-315